(19) Europäisches Patentamt
European Patent Office
Office européen des brevets

(11) Veröffentlichungsnummer: **0 343 480**
**A1**

# (12) EUROPÄISCHE PATENTANMELDUNG

(21) Anmeldenummer: **89108707.4**

(22) Anmeldetag: **16.05.89**

(51) Int. Cl.⁴: **A61K 39/39 , A61K 39/29 , A61K 39/00**

(30) Priorität: **27.05.88 DE 3818054**

(43) Veröffentlichungstag der Anmeldung:
**29.11.89 Patentblatt 89/48**

(84) Benannte Vertragsstaaten:
**AT BE CH DE ES FR GB GR IT LI LU NL SE**

(71) Anmelder: **BIOTEST PHARMA GMBH**
**Landsteiner Strasse 5**
**D-6072 Dreieich(DE)**

(72) Erfinder: **Meuer, Stefan, Prof.Dr.med.**
**Posselstrasse 8**
**D-6900 Heidelberg(DE)**
Erfinder: **Schwulera, Udo, Dr.phil.nat.**
**Dipl.-Biol.**
**Krebsbachweg 23**
**D-6450 Hanau 1(DE)**
Erfinder: **Thrun, Alexander Dr.phil.nat.**
**Dipl.-Biol.**
**Konrad-Duden-Weg 39**
**D-6000 Frankfurt/M.56(DE)**
Erfinder: **Lissner, Reinhard, Dr.med,**
**Dipl.-Chem.**
**Gönz 18**
**D-8761 Weilbach(DE)**

(74) Vertreter: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

## (54) Verwendung von Interleukin-2 zur Immunrekonstitution bei abgeschwächter Immunreaktion.

(57) Verwendung einer Kombination aus einem Antigen bzw. einer Vakzine und humanem n IL-2 und/oder r IL-2 in niedrigen Dosierungen zur Rekonstitution einer primären oder sekundären Immundefizienz, vorzugsweise zur Prävention und Therapie bei infektionsbedingten oder parasitären Erkrankungen beim Menschen.

EP 0 343 480 A1

## Verwendung von Interleukin-2 zur Immunrekonstitution bei abgeschwächter Immunreaktion

Die Erfindung betrifft die Verwendung von Interleukin-2 zusammen mit bestimmte Antikörper hervorrufendem Antigen bzw. Vakzine zur Immunrekonstitution bei Menschen.

Interleukin-2 (IL-2), von Morgan et al., Science (1976), 1007, als T-Zellwachstumsfaktor entdeckt und von Taniguchi et al, Nature (1983), 305, charakterisiert, weist neben der obengenannten Wirkung vielfältige andere biologische Funktionen auf, wobei sich IL-2 an seinen Rezeptor anlagern muss und das Signal zum Zellkern über einen noch unbekannten Mechanismus geleitet wird. Zu diesen profilerationsunabhängigen Effekten gehören z.B. die Steigerung der cytotoxischen Aktivität von natürlichen Killerzellen (NK), die Induktion der cytotoxischen Aktivität von Lymphokin-aktivierten Killerzellen (LAK-Zellen), die Differenzierung von Thymozyten zu cytotoxischen T-Lymphozyten oder die Aktivierung der Synthese von Interferon-Gamma und anderen Cytokinen (Fink et al, DMW (1987), 188).

Diese in-vitro und in-vivo nachgewiesenen biologischen Aktivitäten zeigen, dass Interleukin-2 eine zentrale Rolle bei der T-zellabhängigen Immunität spielt und die zelluläre Immunantwort verstärkt. Es ist deshalb nicht zu erwarten, dass es überhaupt einen Einfluss auf das humorale Immunsystem hat.

Aufgrund der obengenannten biologischen Aktivitäten wurde daher IL-2 therapeutisch bisher vorwiegend zur Behandlung von Tumor-Erkrankungen eingesetzt (s. auch S.A. Rosenberg, Immunol. Today (1988), 58). Hierbei führt sowohl der Einsatz von IL-2 allein als auch zusammen mit LAK's zu einem gewissen Prozentsatz zu kompletten oder partiellen Remissionen, wobei jedoch durch die LAK/IL-2 Therapie verursachte starke Nebenwirkungen bis hin zur Letalität auftreten. Darüberhinaus liegt die Gesamtdosierung in der Nähe der maximalen Toleranz. Niedrige Dosierungen von systemisch appliziertem IL-2 scheinen bei onkologischen Patienten wirkungslos zu sein. Seinen indirekten (über die Aktivierung von NK, LAK-Zellen bzw. Thymozyten) cytotoxischen und cytostatischen Effekt kann IL-2 nur bei sehr hohen Konzentrationen entfalten.

Andere Indikationen ausser Neoplasien, bei welchen IL-2 eingesetzt wird, wurden klinisch bisher nicht untersucht. Lediglich im Tierversuch konnte gezeigt werden (EP-A 0 219 979), dass die gleichzeitige Gabe von Vakzinen und IL-2 zur Behandlung bei Immunsuppressionen zu einer Verstärkung der durch das Antigen hervorgerufenen Immunantwort bei Mäusen führt. Voraussetzung für die Effizienz eines solchen Mittels ist hier also das Ansprechen der zu immunisierenden Tiere auf das Antigen per se; reagieren die gesunden, nicht immundefizienten Tiere auf das Antigen per se nicht, ist auch eine IL-2-Gabe nutzlos. IL-2 agiert also als Adjuvanz. Das Mittel wird hierbei intramuskulär verabreicht, wobei eine signifikante Wirkung erst bei relativ hoher Interleukin-2-Dosierung erzielt wird.

Der Erfindung liegt daher die Aufgabe zugrunde, ein Mittel bereitzustellen, das bei abgeschwächter Immunreaktion gegenüber z.B. Virusinfektionen bzw. bei erworbenen oder angeborenen Autoimmunerkrankungen zu einer Immunrekonstitution führt und in solchen Mengen verabreicht werden kann, die zu keinen oder nur sehr geringen Nebenwirkungen führen.

Diese Aufgabe wird erfindungsgemäss dadurch gelöst, dass eine Kombination eines gegen bestimmte Viren, Bakterien, Mikroorganismen und Parasiten bzw. Antigene normalerweise aktiven Impfstoffs mit humanen natürlichen und/oder rekombinantem Interleukin-2 (n IL-2 und/oder r IL-2) eingesetzt wird.

Hierbei zeigte sich überraschenderweise, dass trotz seiner kurzen biologischen Halbwertszeit eine niedrige Konzentration von IL-2 bei immunsupprimierten Patienten eine maximale Wirkung zeigt. Darüberhinaus konnte nachgewiesen werden, dass IL-2 hier nicht als Adjuvanz, also als Verstärker einer schon vorhandenen Immunreaktion agiert. Vielmehr wird durch die IL-2 Gabe erst ermöglicht, dass überhaupt eine Immunantwort auf das verabreichte Antigen auftritt. Durch aktives Impfen wird hier also eine Immunabwehr aufgebaut, indem die bei immundefizienten Patienten vorliegenden Blockaden oder der Ausfall von relevanten Botenstoffen umgangen wird. Mit Hilfe des erfindungsgemässen Mittels erfolgt also eine Immunrekonstitution. Das IL-2 wirkt also auf das humorale System.

Im erfindungsgemässen Mittel können das Interleukin-2 und das Antigen bzw. die Vakzine zusammen oder in getrennten Phasen vorliegen. Beide Phasen können in beliebiger Reihenfolge nacheinander verabreicht werden, sie können jedoch auch als Kombination zur Anwendung kommen. Die Präparate können in flüssiger Form oder als Lyophilisat vorliegen, das vorher zu lösen ist oder an einen Träger gebunden bzw. in Kapseln eingeschlossen ist.

Erfindungsgemäss können somit Patienten mit abgeschwächtem Immunsystem, die nicht auf Vakzine bzw. spezielle Antigene ansprechen, präventiv bzw. therapeutisch gegen keim-induzierte Erkrankungen bzw. bei Autoimmun- (d.h. primäre und sekundäre Immundefizienz) oder endokrine Erkrankungen behandelt werden. Solche viral bedingte Infekte sind z.B. Erkrankungen, welche durch Hepatitis B, Hepatitis A, Non A - Non B, andere noch unbekannte Hepatitis-Viren, Papillome, Röteln, Masern, Herpes, Papova (Polyoma, Varizellen), Adenovirus induzierte Krankheiten und andere verursacht werden.

Zu den bakteriell bedingten Infekten gehören beispielsweise Haemophilus influenzae. Parasitäre Erkrankungen, die erfindungsgemäss behandelt werden können, sind z.B. Malaria oder Leishmania.

Zu den obengenannten Autoimmunerkrankungen gehören z.B. Lupus erythematodes, rheumatoide Arthritis, Peri-arteriitis, Dermatomyositis, Morbus Crohn, Diabetes Typ I und andere.

Darüberhinaus können erfindungsgemäss auch Kombinationen bestimmter Antigene mit IL-2 bei der Behandlung von Krankheiten eingesetzt werden, bei denen Viren als Auslöser vermutet werden. Hierzu gehören beispielsweise einige Leukämien, Burkitt-Lymphome, Cervix-Carcinome und andere Neoplasien, neurologische Erkrankungen, Multiple Sklerose, Parkinson-Syndrom, Morbus Alzheimer, Amytrophe Lateral Sklerose, bestimmte Formen der Schizophrenie, Depressionen u.a. neurologische Erkrankungen.

Insbesondere erwies sich die erfindungsgemässe Kombination aus Vakzine und IL-2 wirksam bei der Behandlung der Non-Responsivness von Dialysepatienten auf Impfung mit Hepatitis B-Impfstoff. Hierbei zeigte sich, dass bei Verabreichung einer Kombination aus Hepatitis-Antigen (HBs-Antigen) und IL-2, welche sowohl gleichzeitig, vorzugsweise aber nacheinander, erfolgen kann, die dialysepflichtigen Patienten, die auf dieses Antigen zuvor nicht mit Antikörperbildung reagierten, überraschenderweise Antikörper gegen das HBs-Antigen bilden. Dabei wurden keinerlei Nebenwirkungen beobachtet.

Die Erfindung wird anhand der folgenden Beispiele näher erläutert:

## I. T-Zell-Proliferationsmessungen in vitro bei Respondern und Non-Respondern

Wie schon seit langem bekannt (The Lancet (1985), 1412) reagieren dialysepflichtige Non-Responder allgemein schwach auf antigene Reize bezüglich der zellvermittelten Immunantwort.
Beispielsweise reagieren viele Dialysepatienten auf eine Hepatitis B-Impfung nicht mit Antikörperbildung gegen das HBs-Antigen des Hepatitis B-Virus (Non-Responder bzw. Weak(Schwacher)-Responder). Durch aktives Impfen lässt sich hier also keine ausreichende Immunabwehr aufbauen, da im Immunsystem des Behandelten Blockaden vorliegen oder Ausfälle von Botenstoffen auftreten.

Anhand der folgenden Untersuchungen wird gezeigt, welche Art von Blockade vorzuliegen scheint und wie diese aufzuheben ist, so dass eine Immunrekonstitution erfolgt. Hierzu wurde die T-Zell-Proliferation in vitro von T-Zellen gesunder bzw. dialysepflichtiger Non-Responder im Hinblick auf Hepatitis B-Antikörperbildung hin untersucht (vgl. St.C.Meuer et al, J.Clin.Inv. (1987), 743).

### Beispiel 1: Gewinnung von T-Zell-Populationen und Monozyten

Den zu behandelnden dialysepflichtigen Patienten wurde zunächst hepariniertes Blut entnommen. Durch Dichtezentrifugation über Ficoll-Paque von Pharmacia (Freiburg) erhielt man periphäre mononukleäre Blutzellen (PBMC). Die Intaktheit dieser Zellen von jedem Patienten wurde mittels indirekter Immunfluoreszenz in einem Zellsorter (Epics-C) gemessen.

T-Zell-Populationen wurden über E-Rosettierung mit Schafs-Erythrozyten (CD 2 x T 11) gewonnen. Anschliessend wurden $E^+$ und $E^-$-Zellen individuell auf Petri-Schalen über Nacht bei 37° C, 6% $CO_2$ in feuchter Atmosphäre gezüchtet. Kulturmedium war RPMI 1640 mit 10% fötalem Kälberserum, 1% Penicillin-Streptomycin und 2% L-Glutamin.

Um restliche Monozyten abzureichern, wurden nicht-adhärente $E^+$-Zellen mit monoklonalen Antikörpern anti J 2 und anti-Mo 2 für 1/2 h bei 37° C behandelt, einmal gewaschen und anschliessend für 1 h bei 37° C im Schüttelwasserbad mit Kaninchen-Serum behandelt. Nach 3 Waschgängen wurden die Zellen auf 2 x $10^6$ Zellen/ml eingestellt und im Standard-Medium über Nacht inkubiert. Diese Zellen waren die Quelle für gereinigte, ruhende T-Zellen (T-Zellen, die keinen Antigen-Kontakt haben und keinerlei immunphysiologische Aktivitäten zeigen).

Die gereinigten T-Zellen wurden zur Überprüfung der Reinheit und der intakten Physiologie mit den monoklonalen Antikörpern anti-T 11 (T-Zell-spezifisch) und anti-MO 2 (Monozyten-spezifisch) behandelt. Die anti-T 11 Aktivität war im Mittel >96%, meistens aber >99%. Die anti-MO 2-Aktivität war immer negativ. Adhärente E-Zellen wurden vorsichtig mit einem Zell-Rechen (Fa. Nunc) von der Petri-Schale entfernt und dienten als Quelle für Monozyten. Die so isolierten Zellen waren zu mehr als 90% lebensfähig (Überprüfung nach der Trypan-Blau-Methode).

### Beispiel 2: Proliferation der ruhenden T-Zellen nach Zugabe verschiedener Stimuli in Anwesenheit bzw.

Abwesenheit von adhärenten Zellen (Monozyten)

Die wie oben beschrieben isolierten ruhenden T-Zellen von Non-Respondern wurden mit anti-T 3-Sepharose-gekoppelten Antikörpern, anti-T 11, PHA-P aktiviert.

Hierzu wurden 1 x $10^5$ Responder T-Zellen in Abwesenheit bzw. in Gegenwart von 5% Monozyten (zuvor mit 6000 rad bestrahlt, d.h. diese Zellen wirken nur noch als Antigen) in 200 $\mu$l Kulturmedium (RPMI 1640, 10% fötales Kälberserum, 1% Penicillin-Streptomycin und 2% L-Glutamin) in Mikrotiter-Platten mit Rundboden mit den obengenannten Stimuli inkubiert. Nach 3 Tagen in vitro-Kultur wurden die Zellen in der Mikrotiterplatte mit $^3$H-Thymidin 16 Stunden markiert.

Anschliessend wurde die $^3$H-Thymidin-Aufnahme in die T-Zellen im Flüssig-Szintillations-Spektrometer der Firma Packard gemessen und mit dem $^3$H-Thymidineinbau bei T-Zellen gesunder Kontrollpersonen verglichen.
Die Ergebnisse sind in Tabelle 1 angegeben. Hierbei sind alle Werte Mittelwerte von jeweils 3 Messungen.

Tabelle 1

| Responder -Zellen | Stimulus | Pat.4 | Pat.5 | Pat.13 | Pat.10 | Pat.19 | Pat.23 | I* | II* |
|---|---|---|---|---|---|---|---|---|---|
| T Zellen | Medium | 246 | 624 | 707 | 544 | 481 | 374 | 320 | 916 |
| $^{T}$ Zellen | S-anti-T3 | 332 | 290 | 1.030 | 640 | 1.572 | 910 | 933 | 1.211 |
| $^{T}$Patient$^{+MØ}$Patient | Medium | 379 | 622 | 756 | 243 | 55 | 289 | 789 | 245 |
| $^{T}$Patient$^{+MØ}$Patient | S-anti-T3 | 1.569 | 2.214 | 6.963 | 8.304 | 23.731 | 15.123 | 15.123 | 23.855 |
| $^{T}$Patient$^{+MØ}$ Kontrolle | S-anti-T3 | 20.961 | 14.466 | 19.136 | 18.894 | 22.457 | 16.894 | 14.987 | 24.888 |
| $^{T}$ Kontrolle$^{+MØ}$ Patient | S-anti-T3 | 8.307 | 6.055 | 12.194 | 13.794 | 25.337 | 30.000 | 15.123 | 24.888 |

* Bei gesunden Kontrollpersonen wurden autologe T-Zellen und Monozyten (MØ) kombiniert.
S-anti-T3 = an Sepharose gekoppelter anti-T3-Antikörper
Medium = RPMI 1640, 10% fötales Kälberserum, 1% Penicillin-Streptomycin, 2% L-Glutamin
MØKontrolle = Monozyten gesunder Spender
T-Kontrolle = T-Zellen gesunder Spender

Hieraus wird ersichtlich, dass ruhende T-Zellen eines Non-Responders auf anti-T 3-Sepharose-gekoppelte Antikörper nicht ansprechen, wenn diese T-Zellen Kontakt mit autologen Monozyten haben. Die T-Zellen des Non-Responders wurden also durch autologe Monozyten des Non-Responders an der IL-2 Produktion und/oder IL-2-Sekretion gehindert. Die ruhenden T-Zellen selbst lassen sich durch exogen zugeführte Stimuli gut aktivieren. Darüberhinaus sprechen die ruhenden T-Zellen auch auf anti T 11-Antikörper an, d.h. der normale Weg der T-Zellaktivierung über anti T-3 wird hier umgangen und der alternative Weg eingeschlagen. Insgesamt kann hieraus also entnommen werden, dass die unzureichende Immunabwehr bei (dialysepflichtigen) Non-Respondern auf einer Blockade auf Seite der Monozyten beruht.

Beispiele 3: T-Zell-Proliferation nach Zugabe von r IL-2 bzw. n IL-2

$10^3$ gereinigte T-Zellen des Non-Responders wurden mit 5x$10^3$ bestrahlten Monozyten inkubiert und rekombinantes IL-2 mit einer Endkonzentration von 2 ng/ml zugegeben. Anschliessend wurde die T-Zell-Proliferation als$^3$H-Thymidineinbau gemessen.

Die Ergebnisse sind in Tabelle 2 aufgezeigt.

Tabelle 2

| Responder -Zelllen | Stimulus | Additiv | Pat. 5 | Pat. 6 | Pat. 19 |
|---|---|---|---|---|---|
| $^{T}$Patient$^{+MØ}$ atient | Medium | Medium | 1.895 | 1.192 | 1.419 |
| $^{T}$Patient$^{+MØ}$Patient | S-anti-T3 | Medium | 2.214 | 4.898 | 23.731 |
| $^{T}$Patient$^{+MØ}$ Kontrolle | S-anti-T3 | Medium | 13.466 | 28.192 | 22.457 |
| $^{T}$Patient$^{+MØ}$Patient | Medium | r IL 2 | 2.669 | 2.957 | 2.158 |
| $^{T}$Patient$^{+MØ}$Patient | S-anti-T3 | r IL 2 | 27.345 | 36.089 | 29.523 |

Wie hieraus zu entnehmen ist, wird mit IL-2, das exogen dem in vitro-Ansatz zugesetzt wird, die T-Zell-Proliferation in Gegenwart von Monozyten erhöht. Dabei werden Werte erreicht, wie sie auch bei T-Zellen von Gesunden zu beobachten sind, wenn diese durch anti-T-3-Sepharose-gekoppelte Antikörper aktiviert werden.

II. Immunrekonstitution in vivo

Im folgenden wurden 10 Dialysepatienten anstelle des anti T 3-Sepharose-gekoppelten Antikörpers als antigener Stimulus mit einer Kombination aus Hepatitis B-Vakzine/IL-2 behandelt, um die Blockade bei der Immunabwehr zu umgehen und eine wirksame Immunrekonstitution zu erzielen.

Beispiel 4: Messung von Antikörper-Titern gegen das HBs-Antigen als Grad für das Ausmass der Immunrekonstitution nach Gabe von Hepatitis B-Vakzine/n IL-2

10 Patienten wurde als antigener Stimulus eine Hepatitis B-Vakzine HB-Vax - eine aus Spenderblut isolierte Vakzine, die normalerweise in Gesunden Antikörper gegen das Oberflächenantigen des Hepatitis B-Virus erzeugt (anti-HBs-Ag-Antikörper) verabreicht.

Der Stimulus wurde - wie es bei der regulären Impfung auch geschieht - zu den Zeitpunkten 0, 1 und 6 Monate verabreicht (40 µg Impfstoff jeweils intraglutäal appliziert). Vier Stunden nach der Application der Vakzine wurde direkt neben der gleichen Einstichstelle n IL-2 appliziert (2,5 x $10^5$ U/ml; pro Applikation 1 ml nIL-2; insgesamt in den 6 Monaten also 7,5 x $10^5$ U nIL-2).

Schema der Applikation

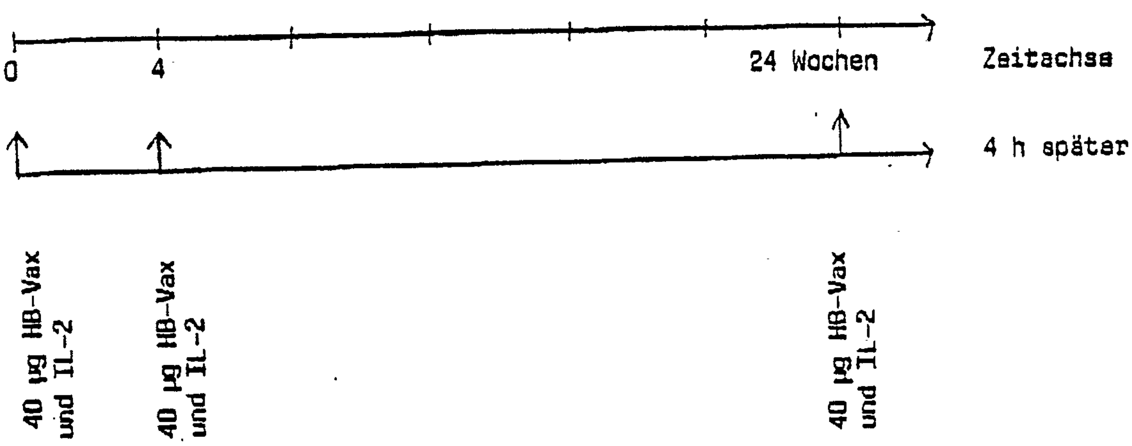


Vom Zeitpunkt 0 an bis 5 Wochen nach der zweiten Vakzine/n IL-2-Gabe wurden die anti-HBs-Antigen-Antikörper-Titer der Patienten gemessen. Überraschenderweise zeigte sich, dass dialysepflichtige Non-Responder erstmalig auf den antigenen Stimulus reagierten. Die Ergebnisse sind in Abb. 1 dargestellt.

Wie hieraus zu entnehmen ist, stieg bei 6 der 10 behandelten Patienten (1, 2,3,4,6,9) der Antikörpertiter, was einer Immunrekonstitution gleichkommt, schon nach 14 Tagen an und wies nach 5 Wochen der Behandlung Werte auf, welche denen von gesunden Respondern entsprechen. Ein Patient (10) hingegen reagierte schwächer auf das verabreichte Mittel (Weak-Responder) und bei 3 (Patienten 5, 7, 8) konnte keine Reaktion festgestellt werden. Diese letztgenannten Patienten weisen jedoch kaum (Patient 10) bzw. keine (Patienten 5, 7, 8) IL-2-Rezeptoren auf, so dass die Zugabe von n IL-2 nicht wirksam sein kann.

Interessanterweise stiegen andere Antikörpertiter (Toxoplasmose, Cytomegalie u.a.) nicht an, d.h. der antigene Stimulus (Hepatitis-Vakzine) wirkte im Zusammenhang mit dem humanen n IL-2 trotz der wesentlich komplexeren Situation in vivo so wie man es in in vitro-Experimenten gesehen hatte. IL-2 ist also ein zentraler Immunregulator, mit dem man offensichtlich schwerwiegende Immundefizienzen im menschlichen Körper im Sinne einer Immunrekonstitution gezielt beheben kann. Es bietet sich somit an, Krankheitsbilder, die auf einer ähnlichen Defizienz beruhen, mit IL-2 in der oben aufgeführten Weise zu beheben.

Beispiel 5: Antikörper-Titer gegen das HBs-Antigen nach Gabe einer Hepatitis B-Vakzine bei Non-Respondern ohne IL-2 Gabe

5 der im vorgenannten Beispiel behandelten Patienten wurden in den Jahren 1980-1987 jährlich mit einer Hepatitis B-Vakzine (20 bzw. 40 µg) behandelt, um eine aktive Immunisierung gegen Hepatitis B zu bewirken.

Die Ergebnisse sind in Abb. 2 zu sehen.

Keiner der Patienten entwickelte anti-HBs-Antigen-Antikörper.

III. Untersuchung der Adjuvanzwirkung von Interleukin-2

Wie vorstehend in der EP-A 0 219 979 sowie von Th.Merigan et al., J. Immunolog. (1988), 294, erwähnt, kann IL-2 zumindest im Tierversuch adjuvant wirken, d.h. die immunogene Wirkung des Antigens, dem es beigemischt wird verstärken.

Bekannte Adjuvantien sind Aluminiumverbindungen, Mineralöle sowie inaktivierte Mykobakterium. Der adjuvante Effekt beruht bei diesen Substanzen auf einer Makrophagen-Membranaktivierung (Verstärkung der Antigenpräsentation), Granulombildung, einer Depotwirkung (verzögerte Freisetzung des Antigens), einer schnellen Verteilung des Antigens über die Lymphbahnen in die Lymphknoten, einer Verstärkung der Primärantwort (gesteigerte Immunogenität).

Im nachfolgenden Beispiel 6 wird nachgeprüft, ob IL-2 tatsächlich eine solche Adjuvanzwirkung ausübt oder vielmehr eine Immunrestauration (wie schon in den vorangegangenen Beispielen 2-4 gezeigt) hervorruft.

Beispiel 6: Messung des Botulinus-toxin-Antikörpertiters in Mäusen nach Zugabe bekannter Adjuvantien bzw. IL-2

5 Gruppen (je 10 männliche BALB/c-Mäuse) wurden mit je 5 µg detoxifiziertem Botulinustoxin A mit und ohne Adjuvanz immunisiert.

| Gruppe | Antigen mit/ohne Adjuvanz |
|---|---|
| 1 | Botulinustoxin A |
| 2 | Botulinustoxin A mit Aluminiumhydroxid |
| 3 | Botulinustoxin A mit IL-2 |
| 4 | Botulinustoxin A mit IL-2 und Aluminiumhydroxid |
| 5 | Botulinustoxin A mit komplettem Freud'schen Adjuvanz |

Die IL-2-Endkonzentration pro Tier lag bei ca.10 000 U.

Der Antikörpertiter gegen das inaktivierte Botulinustoxin A wurde an den Tagen 0, 13, 27 und 38 mit Hilfe eines ELISA bestimmt.

Die Ergebnisse sind in Abb. 3 dargestellt. Hierbei bedeuten die angegebenen Werte jeweils Mittelwerte aus 10 Einzelbestimmungen pro Gruppe.

Die Ergebnisse zeigen, dass die höchsten Titer am Tag 13 bei Zusatz von Freud'schen Adjuvanz (Gruppe 5) beobachtet werden. Auch die Zugabe von Aluminiumhydroxid mit und ohne IL-2 (Gruppe 2 und 4) bewirkt einen hohen Antikörpertiter, während IL-2 alleine (Gruppe 3) keinen bzw. sogar einen leicht hemmenden Einfluss auf die Antikörpersynthese hat im Vergleich zu den Tieren der Gruppe 1, die nur das Antigen erhielten.

Hieraus wird klar ersichtlich, dass die zusätzliche Gabe von Interleukin-2 keinen Einfluss bzw. sogar eine hemmende Wirkung auf die Botulinustoxin A Antikörperbildung bei BALB/c-Mäusen hat und somit nicht adjuvant wirkt, d.h. kein Adjuvanz per se ist. Vielmehr muss man sogar davon ausgehen, dass eine exogene IL-2-Zufuhr zu Systemen, die nicht immundefizient sind, eher einen störenden Einfluss, zumindest aber keinen verstärkenden Einfluss auf die Immunantwort ausübt.

Insgesamt kann also durch gleichzeitige Verabreichung eines Impfstoffs bzw. eines bestimmte Antikörper hervorrufenden Antigens und humanen n IL-2 und/oder r IL-2 eine Immunrekonstitution, also keine Adjuvanzwirkung, bei immundefizienten Patienten erzielt werden. Durch die einfache Applikation (intraglutinäal) und die sehr geringen Mengen an IL-2 treten keine störenden Nebenwirkungen auf. Erfindungsgemäss ist es somit erstmals möglich, viral oder durch andere Organismen bedingten Erkrankungen bei geschwächtem Immunsystem bzw. erworbenen oder angeborenen Autoimmunkrankheiten bei immundefizienten Patienten wirksam entgegenzutreten.

## Ansprüche

1. Verwendung einer Kombination aus einem Antigen bzw. einer Vakzine und humanem Interleukin-2 in Form von n IL-2 und/oder r IL-2 zur präventiven bzw. therapeutischen Rekonstitution der Immunabwehr gegen Infekte bei geschwächten Immunsystemen und/oder erworbenen oder angeborenen Autoimmunkrankheiten beim Menschen.

2. Verwendung nach Anspruch 1, dadurch gekennzeichnet, dass das Mittel in einer Verpackungseinheit konfektioniert ist und aus zwei räumlich getrennten Zusammensetzungen besteht, von denen die eine Phase das Antigen bzw. die Vakzine und die andere Phase das humane Interleukin-2 in Form von n IL-2 und/oder r IL-2 enthält und dass die beiden Phasen gleichzeitig oder beliebig aufeinanderfolgend angewendet werden.

3. Verwendung nach Anspruch 1 oder 2 zur Rekonstitution der Immunabwehr gegen Hepatitis-B-Antigen bei dialysepflichtigen Patienten.

Patentansprüche für folgenden Vertragsstaat:ES

1. Verfahren zur Herstellung eines Mittels zur präventiven bzw. therapeutischen Rekonstitution der Immunabwehr gegen Infekte bei geschwächten Immunsystemen und/oder erworbenen oder angeborenen Autoimmunkrankheiten beim Menschen, dadurch gekennzeichnet, dass man ein Antigen bzw. eine Vakzine und humanes Interleukin-2 in Form von n IL-2 und/oder r IL-2 kombiniert.

2. Verfahren gemäss Anspruch 1, dadurch gekennzeichnet, dass man das Mittel in einer Verpackungseinheit so konfektioniert, dass es aus zwei räumlich getrennten Zusammensetzungen besteht, wobei die eine Phase das Antigen bzw.die Vakzine und die andere Phase das humane Interleukin-2 in Form von n IL-2 und/oder r IL-2 enthält und die beiden Phasen gleichzeitig oder beliebig aufeinanderfolgend angewendet werden können.

3. Verfahren gemäss Anspruch 1 oder 2 zur Rekonstitution der Immunabwehr gegen Heptatitis-B-Antigen bei dialysepflichtigen Patienten.

4. Verwendung einer gemäss den Ansprüchen 1-3 hergestellten Kombination aus einem Antigen bzw. einer Vakzine und humanen Interleukin-2 in Form von n IL-2 und/oder r IL-2 zur präventiven bzw. therapeutischen Rekonstitution der Immunabwehr gegen Infekte bei geschwächten Immunsystemen und/oder erworbenen oder angeborenen Autoimmunkrankheiten beim Menschen.

5. Verwendung nach Anspruch 4, dadurch gekennzeichnet, dass das Mittel in einer Verpackungseinheit konfektioniert ist und aus zwei räumlich getrennten Zusammensetzungen besteht, von denen die eine Phase das Antigen bzw. die Vakzine und die andere Phase das humane Interleukin-2 in Form von n IL-2 und/oder r IL-2 enthält und dass die beiden Phasen gleichzeitig oder beliebig aufeinanderfolgend angewendet werden.

6. Verwendung nach Anspruch 4 oder 5 zur Rekonstitution der Immunabwehr gegen Hepatitis-B-Antigen bei dialysepflichtigen Patienten.

Abb. 1

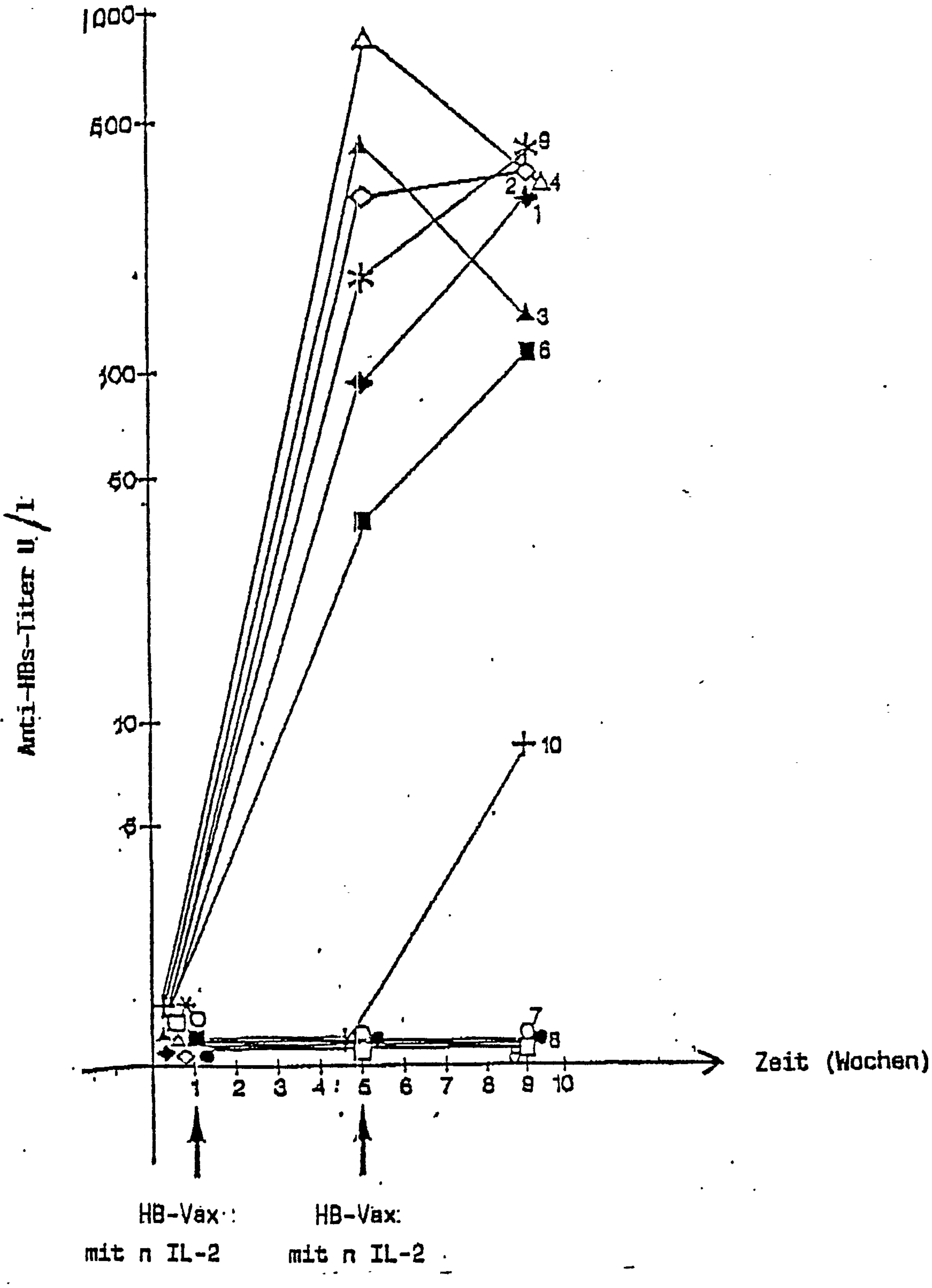
1000
500
100
50
10
5
Anti-HBs-Titer U/l
Zeit (Wochen)
1 2 3 4 5 6 7 8 9 10
9
2
4
1
3
6
10
7
8
HB-Vax:
mit n IL-2
HB-Vax:
mit n IL-2

Abb. 2

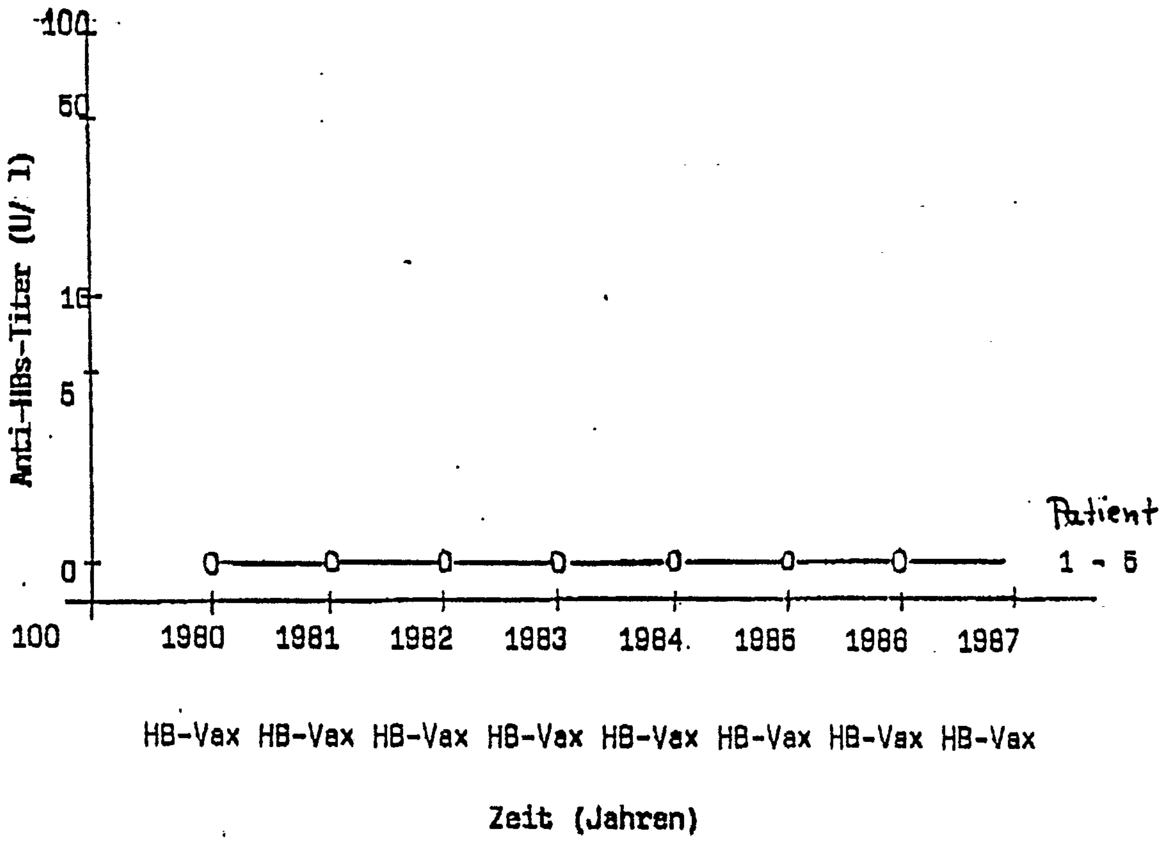
100
50
10
5
0
Anti-HBs-Titer (U/ l)
Patient
1 - 5
100
1980
1981
1982
1983
1984
1985
1986
1987
HB-Vax HB-Vax HB-Vax HB-Vax HB-Vax HB-Vax HB-Vax HB-Vax
Zeit (Jahren)

Abb. 3

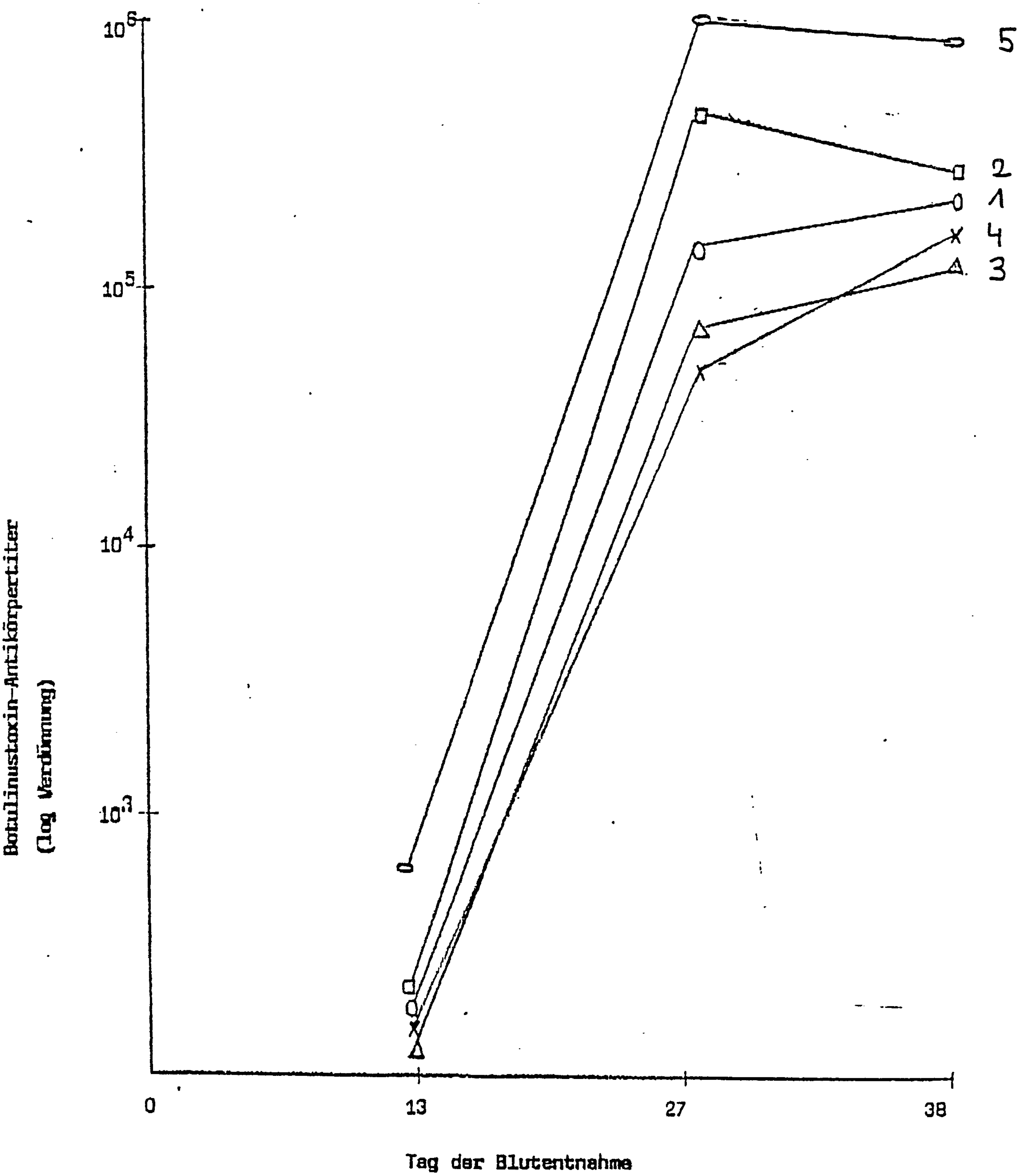

10^6
10^5
10^4
10^3
Botulinustoxin-Antikörpertiter
(log Verdünnung)
0
13
27
38
Tag der Blutentnahme
5
2
1
4
3

Europäisches Patentamt

# EUROPÄISCHER RECHERCHENBERICHT

Nummer der Anmeldung

EP 89 10 8707

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,D | EP-A-0 219 979 (CETUS CORP.)<br>* Ansprüche 1-6 *<br>--- | 1,2 | A 61 K 39/39<br>A 61 K 39/29<br>A 61 K 39/00 |
| X | J. CLIN. LAB. IMMUNOL., Band 26, 1988, Seiten 25-27, Teviot-Kimpton Publications, Edinburgh, GB; S. KAKUMU et al.: "Enhancement of antibody production to hepatitis B surface antigen by interleukin 2"<br>* Seite 26:"Results" *<br>--- | 1 | |
| P,X | EP-A-0 285 441 (INTERNATIONAL MINERALS AND CHEMICAL CORP.)<br>* Ansprüche 1,2,19,20; Seite 4, Zeilen 38-44; Seite 6, Zeilen 3-10 *<br>----- | 1-3 | |
| | | | **RECHERCHIERTE SACHGEBIETE (Int. Cl.4)** |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 08-09-1989 | PEETERS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)